# EUROPÄISCHE PATENTSCHRIFT

(11) **EP 0 829 470 B1**
(45) Veröffentlichungstag und Bekanntmachung des Hinweises auf die Patenterteilung: **15.12.1999**
(21) Anmeldenummer: 97115520.5
(22) Anmeldetag: 08.09.1997
(51) Int. Cl.: C07C 255/23, C07C 255/41, C07D 213/57, C07D 307/54, C07D 333/24, C07D 317/60, C07D 473/04

(54) **2-Cyano-3,5-dihydroxy-hex-2-en-carbonsäure-amidderivate, Verfahren zu ihrer Herstellung und ihre Verwendung als Arzneimittel**
2-Cyano-3,5-dihydroxy-hex-2-ene carboxamide derivatives, process for their preparation and their use as medicaments
Dérivés de 2-cyano-3,5-dihydroxy-hex-2-ene carboxamide, procédé pour leur préparation et leur utilisation comme médicaments

(30) Priorität: 12.09.1996 DE 19636974
(43) Veröffentlichungstag der Anmeldung: 18.03.1998
(73) Patentinhaber: HOECHST AKTIENGESELLSCHAFT, 65929 Frankfurt am Main (DE)
(72) Erfinder: Schwab, Wilfried, Dr., 65207 Wiesbaden (DE); Raiss, Ruth, Dr., 60322 Frankfurt (DE)

(56) Entgegenhaltungen:
- EP-A- 0 652 214
- WO-A-94/24095
- CHEMICAL ABSTRACTS, vol. 109, no. 13, 26.September 1988 Columbus, Ohio, US; abstract no. 109846a, A. YU SIZOV ET AL: "Acylation of cyanoacetic ester with diacid chlorides under phase transfer catalysts" Seite 567; XP002048438 & IZV. AKAD. NAUK SSSR, SER. KHIM., Nr. 11, 1987, Seiten 2604-2606,

## Beschreibung

Die Erfindung betrifft 2-Cyano-3,5-dihydroxy-hex-2-en-carbonsäureamidderivate, Verfahren zu ihrer Herstellung und Verwendung derselben als Arzneimittel.

Hyaliner Gelenk-Knorpel ist ein elastisches Stütz- und Gleitgewebe, dessen biomechanische Funktion durch die spezielle Architektur seiner Matrix sowie deren geregelte Erneuerung seitens der Knorpelzellen oder Chondrozyten gewährleistet wird. Degenerative Gelenkserkrankungen wie Arthrosen, aber auch entzündlich, immunologisch, oder stoffwechselbedingte Arthritiden und Arthropathien sind gekennzeichnet durch eine progrediente Knorpelzerstörung, die über Funktionsbeeinträchtigung und Schmerz bis hin zur völligen Gelenkversteifung führen kann. Wenn auch für die verschiedenen Krankheitsformen unterschiedliche Auslöser dafür verantwortlich gemacht werden, so ist dem resultierenden Knorpelverlust nach allgemeiner Lehrmeinung doch gemeinsam, daß er mit verstärktem Proteoglykan-Abbau beginnt und von den Chondrozyten gesteuert wird.

Herkömmliche Therapeutika dieser Erkrankungen sind für degenerative Gelenkserkrankungen vor allem nicht steroidale Antiinflammatorika, sowie für autoimmunologisch geprägte Arthritiden sogenannte Basistherapeutika wie Goldverbindungen, Penicillamin, Chloroquin-Derivate und Methotrexat bzw. Kombinationen derselben, allen ist jedoch gemeinsam, daß sie die fortschreitende Knorpelzerstörung nicht aufzuhalten vermögen.

Arthrose ist eine degenerative Gelenkerkrankung mit entzündlichen Episoden und progredienter Knorpelzerstörung, die zu Funktionsbeeinträchtigung bis hin zu völliger Versteifung führen kann. Bisher sind zwar die begleitenden Entzündungen und Schmerzzustände bei dieser Erkrankung therapierbar, es stehen aber keine Arzneimittel zur Verfügung, die erwiesenermaßen den fortschreitenden Knorpelabbau aufzuhalten oder zu heilen vermögen. Bekannte Therapeutika der Arthrose sind beispielsweise Mischungen von sulfatierten Glucoseaminoglykanen (Current Therapeutic Research, 40,6 (1986) 1034) oder nicht steroidale Antiinflammatorika, die jedoch den Knorpelverlust nicht aufzuhalten vermögen.

Wenn auch die Pathogenese der Arthrose noch nicht im Einzelnen aufgeklärt ist, gilt als gesichert, daß die Chondrozyten (Knorpelzellen) maßgeblich an dem verstärkten Matrixverlust beteiligt sind, und daß von den Hauptbestandteilen dieser Matrix vor allem die Proteoglykane (PG) als erste enzymatisch abgebaut werden.

Es wurde nun gefunden, daß die erfindungsgemäßen Verbindungen der Formel I die Proteoglykansynthese der Knorpelzelle entweder direkt stimuliert oder den durch Interleukin-1-induzierten verstärkten Proteoglykanabbau hemmt.

Aufgrund ihrer pharmakologischen Eigenschaften eignen sich die erfindungsgemäßen Verbindungen hervorragend zur Behandlung und Prophylaxe von degenerativen Gelenkserkrankungen, aber auch von Erkrankungen des rheumatischen Formenkreises, bei denen Knorpelabbau zu verzeichnen ist, wie bei der chronischen Polyarthritis, dem Gelenktrauma sowie bei Knorpelschwund nach längerer Immobilisation des Gelenks.

Die Erfindung betrifft eine Verbindung der Formel I 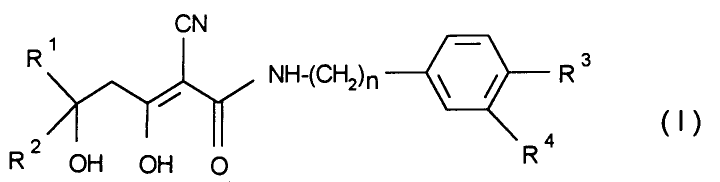 und/oder eine gegebenenfalls stereoisomere Form der Verbindung der Formel I und/oder ein physiologisch verträgliches Salz der Verbindung der Formel I, wobei
R¹ für
   a) Wasserstoffatom oder
   b) (C₁-C₄)-Alkyl steht,
R² für
   a) (C₁-C₁₂)-Alkyl,
   b) (C₂-C₁₂)-Alkenyl,
   c) (C₂-C₁₂)-Alkinyl,
   d) (C₃-C₇)-Cycloalkyl,
   e) ein 4- bis 7-gliedriger heterozyklischer Rest mit 1 oder 2 Heteroatomen aus der Gruppe Sauerstoff, Stickstoff und Schwefel
   f) Phenyl,
   g) Phenyl, ein- bis dreifach substituiert durch
      1) (C₁-C₄)-Alkyl,
      2) Halogen,
      3) -CN,
      4) -CF₃,
      5) -O-(C₁-C₄)-Alkyl,
      6) -O-(C₁-C₄)-Alkyl substituiert durch Phenyl, oder
      7) Methylendioxyl,
   h) (C₁-C₆)-Alkyl, einmal substituiert durch
      1) Phenyl oder
      2) Rest der Formel II, 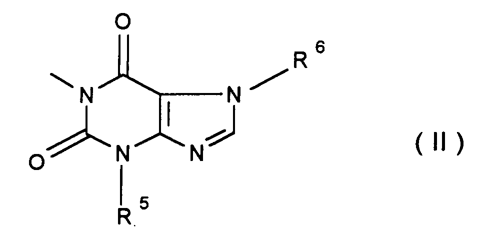 worin R⁵ und R⁶ unabhängig voneinander für Wasserstoffatom oder (C₁-C₄)-Alkyl stehen,
   i) (C₂-C₆)-Alkenyl, einmal substituiert durch Phenyl oder Rest der Formel II, oder
   k) (C₂-C₆)-Alkinyl, einmal substituiert durch Phenyl oder Rest der Formel II, steht, oder
   l) R¹ und R² zusammen = 0 bedeuten,
R³ für
   a) -CF₃,
   b) -O-CF₃,
   c) -S-CF₃,
   d) -OH,
   e) -NO₂,
   f) Halogen,
   g) Benzyl,
   h) Phenyl,
   i) -O-Phenyl,
   j) Wasserstoffatom,
   k) -CN,
   l) -O-Phenyl, ein oder mehrfach substituiert durch
      1) (C₁-C₄)-Alkyl,
      2) Halogen,
      3) -O-CF₃ oder
      4) -O-CH₃, steht,
R⁴ für
   a) (C₁-C₄)-Alkyl
   b) Halogen, oder
   c) Wasserstoffatom steht, und
n für die ganze Zahl Null, 1, 2, 3 oder 4 steht.

Bevorzugt ist eine Verbindung der Formel I und/oder ein physiologisch verträgliches Salz der Verbindung der Formel I und/oder eine gegebenenfalls stereoisomere Form der Verbindung der Formel I, wobei
R¹ für Wasserstoffatom oder Methyl, steht,
R² für
   a) (C₁-C₄)-Alkyl,
   b) (C₂-C₇)-Alkenyl,
   c) Cyclopropyl,
   d) Phenyl,
   e) ein 5- bis 6-gliedriger heterozyklischer Rest aus der Gruppe
      1) Pyridin,
      2) Furan oder
      3) Thiophen,
   f) Phenyl, ein- bis dreifach substituiert durch
      1) Methyl,
      2) -CN,
      3) -CF₃,
      4) -O-Methyl,
      5) -O-Methylphenyl oder
      6) Methylendioxyl,
   g) (C₁-C₄)-Alkyl substituiert durch den Rest der Formel II, worin R⁵ und R⁶ unabhängig voneinander für Methyl oder Propyl stehen,
   h) Vinyl, substituiert durch Phenyl, oder
   i) Ethinyl, substituiert durch Phenyl, steht, oder
   k) R¹ und R² zusammen = 0 bedeuten,
R³ für
   a) -CF₃,
   b) -Cl oder
   c) Methyl steht,
R⁴ für Wasserstoffatom oder Methyl steht, und
n für die ganze Zahl Null, 1 oder 2 steht.

Insbesondere bevorzugt ist eine Verbindung der Formel I und/oder ein physiologisch verträgliches Salz der Verbindung der Formel I und/oder eine gegebenenfalls stereoisomere Form der Verbindung der Formel I, wobei
R¹ für Wasserstoffatom oder Methyl steht,
R² für
   a) Methyl,
   b) Butyl,
   c) Vinyl,
   d) 1-Methyl-ethenyl,
   e) 2-Methyl-prop-enyl,
   f) 2,6-Dimethyl-hepta-1,5-dienyl,
   g) Cyclopropyl,
   h) Phenyl,
   i) Phenyl, ein- oder mehrfach substituiert durch
      1) Methylendioxyl,
      2) 4-Methyl,
      3) Benzyloxy,
      4) 4-Trifluormethyl,
      5) Cyano oder
      6) 3,4,5-Trimethoxy,
   k) Furanyl,
   l) Pyridyl,
   m) Thiophenyl oder
   n) Ethinylphenyl steht, oder
   o) R¹ und R² zusammen = 0 bedeuten,
R³ für
   a) -CF₃,
   b) -Cl oder
   c) Wasserstoffatom steht,
R⁴ für Wasserstoffatom oder Methyl steht und
n für die ganze Zahl Null, 1 oder 2 steht.

Unter dem Begriff Alkyl, Alkenyl oder Alkinyl werden Reste verstanden deren Kohlenstoffkette geradkettig, verzweigt oder cyclisch sein kann; die Doppel-oder Dreifachbindungen können mehrfach vorkommen. Cyclische Alkylreste sind beispielsweise 3- bis 7-gliedrige Monocyclen wie Cyclopropyl, Cyclobutyl, Cyclopentyl, Cyclohexyl oder Cycloheptyl. Zu dem Begriff "4- bis 7-gliedriger heterozyklischer Rest mit 1 oder 2 Heteroatomen aus der Gruppe Sauerstoff, Stickstoff und Schwefel" gehören z.B. Reste, die sich von Azetidin, Pyrrol, Pyran, Azepin, Pyrrolin, Pyrrolidin, Pyridin, Piperidin, Imidazol, Pyrimidin, Furan, 1,2-Diazepin, Oxazol, Pyrazin, Piperazin, Isoxazol, Isoxazolin, Morpholin, Thiazol, Isothiazol, Isothiazolidin, Thiomorpholin, Thiopyran oder Thiophen ableiten. Halogen bedeutet Chlor, Brom, Jod oder Fluor.

Geeignete physiologisch verträgliche Salze der Verbindung der Formel I sind beispielsweise Alkali-, Erdalkali- und Ammoniumsalze einschließlich solcher von organischen Ammoniumbasen.

Die Erfindung betrifft auch ein Verfahren zur Herstellung der Verbindung der Formel I und/oder ein physiologisch verträgliches Salz der Verbindung der Formel I und/oder eine gegebenenfalls stereoisomere Form der Verbindung der Formel I, das dadurch gekennzeichnet ist, daß man
A) ein entsprechend substituiertes 5-Methyl-isoxazol-3-carbonsäureamid oder - anilid bei tiefer Temperatur, bevorzugt bei -80 bis -40°C, mit einem Überschuß (ca. 3 Äquivalente) an starken organischen oder bevorzugt metallorganischen Basen wie Butyllithium, tert.-Butyllithium oder Lithiumdiisopropylamid in wasserfreien organischen Lösungsmitteln, wie Diethylether, Tetrahydrofuran oder tert.-Butylmethylether behandelt, wobei neben der baseninduzierten Ringöffnung des Isoxazolringes eine Deprotonierung der Methylgruppe stattfindet und diese deprotonierte Zwischenstufe mit elektrophilen Reagenzien wie Aldehyden, Ketonen oder Kohlendioxid nach entsprechender Aufarbeitung durch Ansäuern und Extraktion zu einer Addition an die Carbonylgruppe im Sinne einer C-C Verknüpfung führt oder
B) eine Carbonsäure, die mit weiteren funktionellen - oder in Form geschützter Vorstufen vorliegenden - Gruppen substituiert sein kann, nach literaturbekannten Verfahren in ein Säurehalogenid, vorzugsweise ein Säurechlorid überführt, und mit der deprotonierten Form eines geeignet substituierten Cyanessigsäureamides oder -anilides im Sinne einer Kondensation umsetzt.

Die Deprotonierung des Cyanessigsäurederivates wird bevorzugt mittels Natriumhydrid in einem aprotischen wasserfreien Lösungsmittel wie Tetrahydrofuran oder Dichlormethan durchgeführt. Deprotonierung und Kondensation verlaufen bevorzugt in einem Temperaturbereich von 0°C bis Raumtemperatur. Die zum Schutz weiterer funktioneller Gruppen gegebenenfalls mitgeführten Schutzgruppen werden danach nach literaturbekannten Verfahren abgespalten. Verfahren B) eignet sich besonders zur Herstellung chiraler Verbindungen, deren Asymmetriezentren im Carbonsäureteil in definierter Chiralität, wie exemplarisch in Beispiel 10 ausgeführt, in Form eines geschützten sekundären oder tertiären Alkohols, mitgeführt werden können, während nach Verfahren A) die chiralen sekundären Alkohole in der Regel als Enantiomerengemische entstehen. Die Herstellung der meist wasserlöslichen und daher besonders für eine intravenöse Applikation geeigneten physiologisch verträglichen Alkali-, Erdalkali- und organischen Ammoniumsalze erfolgt durch Lösen oder Suspendieren der Stammverbindung in einem protischen oder polaren aprotischen Lösungsmittel und Zugabe der Base äquimolar oder im Überschuß, wobei unter Verwendung polarer aprotischer Lösungsmittel wie Wasser oder niederer Alkohole meist eine klare Lösung des entsprechenden Salzes entsteht, das durch Gefriertrocknung, Aufkonzentrieren der Lösung oder Fällung durch Zugabe eines unpolareren Lösungsmittel in eine feste, bevorzugt kristalline Form gebracht werden kann.

Die Erfindung betrifft auch Arzneimittel, gekennzeichnet durch einen wirksamen Gehalt an mindestens einer Verbindung der Formel I 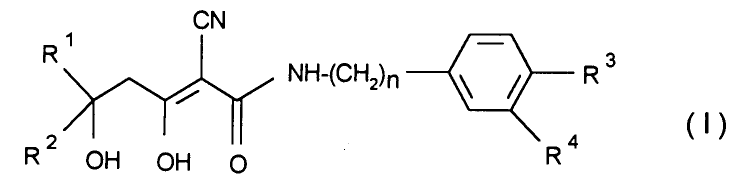 und/oder eines physiologisch verträglichen Salzes der Verbindung der Formel I und/oder eine gegebenenfalls stereoisomere Form der Verbindung der Formel I, wobei die Reste R¹, R², R³ oder R⁴ wie oben definiert sind, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen hervorragend zur Prophylaxe und Therapie all solcher Erkrankungen oder Störungen, an deren Verlauf ein verstärkter Bindegewebs- und Knorpel-Abbau beteiligt ist. Dies sind vor allem Erkrankungen des Bewegungsapparates wie entzündlich, immunologisch oder stoffwechselbedingte akute und chronische Arthritiden und Arthropathien, aber auch Myalgien, Störungen des Knochenstoffwechsels, sowie degenerative Gelenkserkrankungen. Darunter zählen Osteoarthrosen und Spondylosen, aber auch Knorpelschwund nach Gelenk-Trauma oder längerer Ruhigstellung.

Die Erfindung betrifft ferner die Verwendung der Verbindung der Formel I zur Herstellung von Arzneimitteln zur Prophylaxe und Therapie von Erkrankungen des Bindegewebes wie Kollagenosen und peridontalen Gewebsveränderungen.

Die Erfindung betrifft auch ein Verfahren zur Herstellung eines Arzneimittels, das dadurch gekennzeichnet, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit-oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa von 50 bis 300 mg und bei Injektionslösungen in Ampullenform bis zu etwa 300 mg, vorzugsweise aber etwa von 10 bis 100 mg, betragen.

Für die Behandlung eines erwachsenen, etwa 70 kg schweren Patienten sind - je nach Wirksamkeit der Verbindungen gemäß Formel I, Tagesdosen von etwa 20 mg bis 1000 mg Wirkstoff, bevorzugt von 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

### Beispiel 1

### N-(Phenyl)-2-cyano-3,5-dihydroxy-5-methyl-hex-2-en-carbonsäureamid

In 320 ml wasserfreiem Tetrahydrofuran werden unter Schutzgas (Argon) 0,025 Mol (5 g) 4-Phenylaminocarbonyl-5-methyl-isoxazol gelöst. Im Kältebad (-78°C) werden dann 0,08 Mol (32 ml) Butyl-Lithium (2,5 molar in Hexan) zugetropft, wobei ein Temperaturanstieg bis -55°C beobachtet wird. Nach einer Stunde Rühren bei -78°C werden dann 0,13 Mol (7,4 g) absolutes Aceton zugetropft, nach weiteren 1 ½ Stunden bei -78°C, wird mit 20 ml Wasser hydrolysiert und der Ansatz auf etwa 0°C erwärmt. Mit 1 N Salzsäure wird ein pH-Wert von 2 eingestellt, mit Ethylacetat extrahiert, die organische Phase zweimal mit Wasser und einmal mit gesättigter NaCl-Lösung gewaschen, anschließend mit Na₂SO₄ getrocknet und unter vermindertem Druck eingeengt und aus Ethylacetat und Petrolether kristallisiert. Die erhaltenen farblosen Kristalle haben einen Schmelzpunkt von 103°C.
Ausbeute: 3,8 g (58 %)

### Beispiel 10a

### N-(4-Trifluormethyl-phenyl)-2-cyano-3,5-dihydroxy-hex-2-en-carbonsäureamidlysiniumsalz, R-Isomer

### Stufe 1:

### Ethyl-(R)-(-)-3-triisopropylsilyloxy-butyrat

0,077 Mol (10,0 g) Ethyl-(R)-3-hydroxybutyrat werden in 90 ml Dimethylformamid gelöst und 0,151 Mol (10,3 g) Imidazol zugesetzt. Es wird unter Schutzgas im Eisbad auf 0°C abgekühlt und 0,083 Mol (16,05 g) Triisopropylsilylchlorid während 5 min zugetropft. Es wird noch 5 Stunden bei Raumtemperatur gerührt, mit Wasser hydrolysiert, das Produkt mit tert. Butylmethylether extrahiert, die organische Phase mit Na₂SO₄ getrocknet und am Rotationsverdampfer unter vermindertem Druck eingeengt.
Ausbeute: 21,78 g (99,8 %) eines dünnflüssigen Öles.

### Stufe 2:

### (R)-3-Triisopropylsilyloxy-buttersäure

0,0755 Mol (21,78 g) des Produktes von Stufe 1 werden in 1,51 Liter einer 0,01 molarer Lithiumhydroxydlösung in einem Tetrahydrofuran/Wasser 1:1-Gemisch 5 Tage bei 60°C gerührt. Es wird mit wäßriger Zitronensäure angesäuert und mit Ethylacetat das Produkt extrahiert. Die organische Phase wird mit Wasser gewaschen, über Natriumsulfat getrocknet und unter vermindertem Druck eingeengt.
Ausbeute: 12,35 g (63 %) eines öligen Produktes

### Stufe 3:

### (R)-3-Triisopropylsilyloxy-buttersäurechlorid

0,0475 Mol (12,36 g ) des Produktes von Stufe 2 werden in 200 ml absolutem Dichlormethan gelöst und unter Rühren mit 0,0523 Mol (6,64 g) Oxalylchlorid versetzt. Nach 5 Stunden bei Raumtemperatur wird unter vermindertem Druck eingeengt.
Ausbeute: 12,9 g (98 %) eines öligen Produktes.

### Stufe 4:

### N-(4-Trifluormethyl-phenyl)-2-cyano-3-hydroxy-5-(R)-triisopropylsilyloxy-hex-2-en-carbonsäureamid

0,0140 Mol (3,2 g) Cyanessigsäure-4-trifluormethylanilid werden in 125 ml wasserfreiem Tetrahydrofuran gelöst und auf 2 bis 5°C abgekühlt. Der Lösung werden unter Rühren und Schutzgas portionsweise 0,031 Mol NaH (0,93 g, 80 %ig in Mineralöl) zugesetzt, wobei die Temperatur unter 10°C gehalten wird. Es wird dann für 2 Stunden bei Raumtemperatur gerührt (dabei leichte Gasentwicklung). Im Anschluß wird auf 10°C abgekühlt und 0,168 Mol (4,69 g) des Produktes von Stufe 3 portionsweise zugegeben. Bei 15°C wird noch für 20 min gerührt und dann werden 3,2 ml Eisessig zugesetzt, weitere 30 min bei 15°C gerührt, mit 125 ml Eiswasser, das 3,2 ml konzentrierte Salzsäure enthält, versetzt und mit Dichlormethan extrahiert. Die organische Phase wird mit Wasser und gesättigter Natriumchloridlösung gewaschen, mit Natriumsulfat getrocknet und am Rotationsverdampfer eingeengt.
Ausbeute: 9,1 g an Rohprodukt, das an Kieselgel (Ethylacetat/Petrolether,
Gradient: 1/1 bis 12/1) chromatographiert wird. Die Produktfraktionen werden unter vermindertem Druck eingeengt.
Ausbeute 0,72 g eines öligen Produktes.

### Stufe 5:

### N-(4-Trifluormethyl-phenyl)-2-cyano-3,5-dihydroxy-hex-2-en-carbonsäureamid, R-Isomer

0,0015 Mol (0,72 g) des Produktes von Stufe 4 werden in 30 ml 2N wäßrigmethanolischer Salzsäure bei Raumtemperatur gelöst. Nach 4 Tagen wird am Rotationsverdampfer unter vermindertem Druck konzentriert. Der sich dabei abscheidende Niederschlag wird abgesaugt und getrocknet.
Ausbeute: 0,36 g
Schmelzpunkt: 156°C, Drehwert: -18,3° (c=1 in Ethanol).
Die durch HPLC bestimmte Enantiomerenreinheit beträgt > 98 %.

### Beispiel 10b

### N-(4-Trifluormethyl-phenyl)-2-cyano-3,5-dihydroxy-hex-2-en-carbonsäureamidlysiniumsalz, S-Isomer

### Herstellung erfolgte analog zu Beispiel 10a ausgehend von Ethyl-(S)-3-hydroxybutyrat

Schmelzpunkt: 157°C, Drehwert: + 16,9° (c=1 in Ethanol).
Die durch HPLC bestimmte Enantiomerenreinheit beträgt > 98 %.

### Beispiel 37:

### N-(4-Trifluormethyl-phenyl)-2-cyano-3,5-dihydroxy-hex-2-carbonsäureamidlysiniumsalz

0,063 Mol (20,0 g) des Produktes aus Beispiel 9 werden in 1 Liter Wasser suspendiert und durch Zusatz von 0,063 Mol (10,3 g) Lysin x H₂O bei einem pH-Wert von 7,2 in Lösung gebracht. Die Lösung wird filtriert und gefriergetrocknet. Die weitgehend amorphe Substanz hat einen Schmelzpunkt von 135-138°C.
Ausbeute: 27,74 g (96 %).
Die Herstellung der Beispiele 38 und 39 erfolgt analog zu Beispiel 37.

### Pharmakologische Beispiele

In geeigneter Zell- oder Gewebskultur können Knorpelzellen ex vivo ihren charakteristischen Knorpelmatrix-Stoffwechsel, d.h. den geregelten Auf- und Abbau der Matrix-Makromoleküle, über einen Zeitraum von etlichen Wochen bis Monaten aufrechterhalten. Diese Prozesse können chemisch beeinflußt werden. In den nachfolgenden Versuchsbeschreibungen wird zum Einen die Wirkung von Prüfverbindungen auf die normale Erneuerung der Knorpelmatrix-Proteoglykane durch die Chondrozyten beschrieben (A), und zum Anderen die Wirkung auf einen pathologisch gesteigerten Proteoglykanabbau sowie gehemmter Synthese in Chondrozyten bzw. Knorpelgewebe durch Zugabe von Interleukin-1 (B). In beiden Assays wird die stimulierende Wirkung der Prüfsubstanz ausgedrückt durch einen Stimulationsfaktor, der sich errechnet aus dem Quotient des bestimmten Proteoglykangehaltes unter Wirkstoffbedingung dividiert durch den Proteoglykangehalt unter Kontrollbedingung. In Versuch A ist die Kontrollbedingung die unbehandelte Kontrollgruppe, in Versuchsanordnung B ist dies die Interleukin-1 (IL-1)-behandelte Kontrollgruppe. Ein Faktor = 1 läßt somit keine Wirkung erkennen, ein Faktor > 1 weist auf eine Stimulation, ein Faktor < 1 auf eine Hemmwirkung hin.

### A) Modulation des chondrozytären Proteoglykanstoffwechsels (PG-Stimulation)

Aus Fesselgelenken frisch geschlachteter Stiere werden unter sterilen Bedingungen Knorpelproben entnommen, mit 1%iger Pronase (Boehringer)-Lösung in Ham's F12 Medium (Serva) bei 37°C für 1 Stunde inkubiert, die Enzymlösung durch eine 0,025 %ige Collagenase Typ A (Worthington)-Lösung in Medium ersetzt und über Nacht inkubiert. Nach Filtern über 50 µm Nylonfilter, Zentrifugation, Resuspension und Vitalitätsprüfung wird eine Zellsuspension von 4 x 10⁶ Zellen/ml Medium, mit 20 % fötalem Kälberserum angereichert, hergestellt und bei etwa 38°C mit 2 %iger niedrig schmelzender Agarose (Seplaque) im Verhältnis 1:1 gemischt und davon 0,1 ml in jedes Well einer 24 Well-Mikrotiterplatte pipettiert. Nach Gelieren werden die Proben mit 0,5 ml/well Medium überschichtet, das mit 5 % Serum, 25 µm/ml Ascorbinsäure, sowie in Gruppen zu 6-8 Wells mit 10 µm der Prüfsubstanz angereichert ist. Für einen Behandlungszeitraum von 8 Tagen wird das Medium in gleichbleibender Zusammensetzung jeden zweiten Tag erneuert. Am Ende der Behandlung wird mit Medium gewaschen, pro Well 1 ml einer gepufferten (pH 5,6) 1 %igen Alcianblau (Sigma)-Lösung, mit 25 %igem Glutaraldehyd im Verhältnis 7:1 angereichert, zugegeben und für 48 Stunden inkubiert. Nach Waschen und Differenzieren mit 3 %iger Essigsäure wird über die aufsteigende Alkoholreihe bis zu 70 %igem Ethanolgehalt entwässert, und dann der gebundene Farbstoff mit 8 M Guanidinhydrochlorid bei 4°C über 24 Stunden extrahiert. Die stöchiometrische Bindung des Alcianblau an die Chondroitinsulfat-Seitenketten der Proteoglykane erlaubt eine Quantifizierung der angereicherten Matrix über die Bestimmung der Extinktion der Farblösung bei 610 nm. Tabelle 1 zeigt die Ergebnisse.

### B) Modulation der IL-1-induzierten chondrozytären Chondrolyse (Chondrolyse-Kompensation)

Die Zell-Präparation erfolgt wie unter A beschrieben, im Falle des Einsatzes von Knorpel-Gewebsexplantaten werden diese nach Ausstanzen von Knorpelscheiben von 4 mm Durchmesser und einer Höhe, die die gesamte Knorpelschicht umfaßt, direkt in das oben beschriebene angereicherte Kulturmedium gegeben. Nach 5-tägiger Adaptation wird dem Medium Interleukin-1- zugegeben, für die Zellkultur 8 Units/well, für die Gewebskultur 20 Units/well, und über die Behandlungsdauer von 8 Tagen ebenfalls jeden zweiten Tag bei Mediumwechsel erneut zugesetzt. Am Versuchsende wird 1µCi Na₂³⁵SO₄/well zugefügt und über 24 Stunden inkubiert. Nach Entfernen des Überstands werden die verbliebenen Gele bzw. Explanate in den Mikrotiterplatten durch wiederholtes Tieffrieren und Auftauen aufgeschlossen und mit 8 M Guanidinhydrochlorid extrahiert. Nach Zentrifugation wird der Überstand über eine PD10 Sephadex-Säule in freies und inkorporiertes Sulfat getrennt und im β-Counter die Radioaktivität der Proben bestimmt, die ein Maß für die neugebildete Proteoglykanmenge darstellt. Tabelle 1 zeigt die Ergebnisse.

### Hinweis zu Tabelle 1:

Die Beispiele 2-9 und 11-33 wurden analog zu Beispiel 1 unter Verwendung der entsprechend substituierten (käuflichen) Aldehyde bzw. Ketone als elektrophile Reagenzien hergestellt. Zur Herstellung der Beispiele 34 und 35 wurden 3-Methyl-(5-oxohexyl)-7-propylxanthin (Propentofyllin) bzw 1-(5-Oxohexyl)-theobromin (Pentoxifyllin) eingesetzt. Beispiel 36 wurde durch Verwendung von festem CO₂ als Elektrophil synthetisiert.
¹H-NMR-Spektren sind an einem 200-MHz-Gerät der Firma Varian aufgenommen worden, in der Regel mit Tetramethylsilan (TMS) als internem Standard und bei Raumtemperatur 22-26°C (RT). Temperaturangaben in Grad Celsius. Die verwendeten Abkürzungen sind entweder erläutert oder entsprechen den üblichen Konventionen.

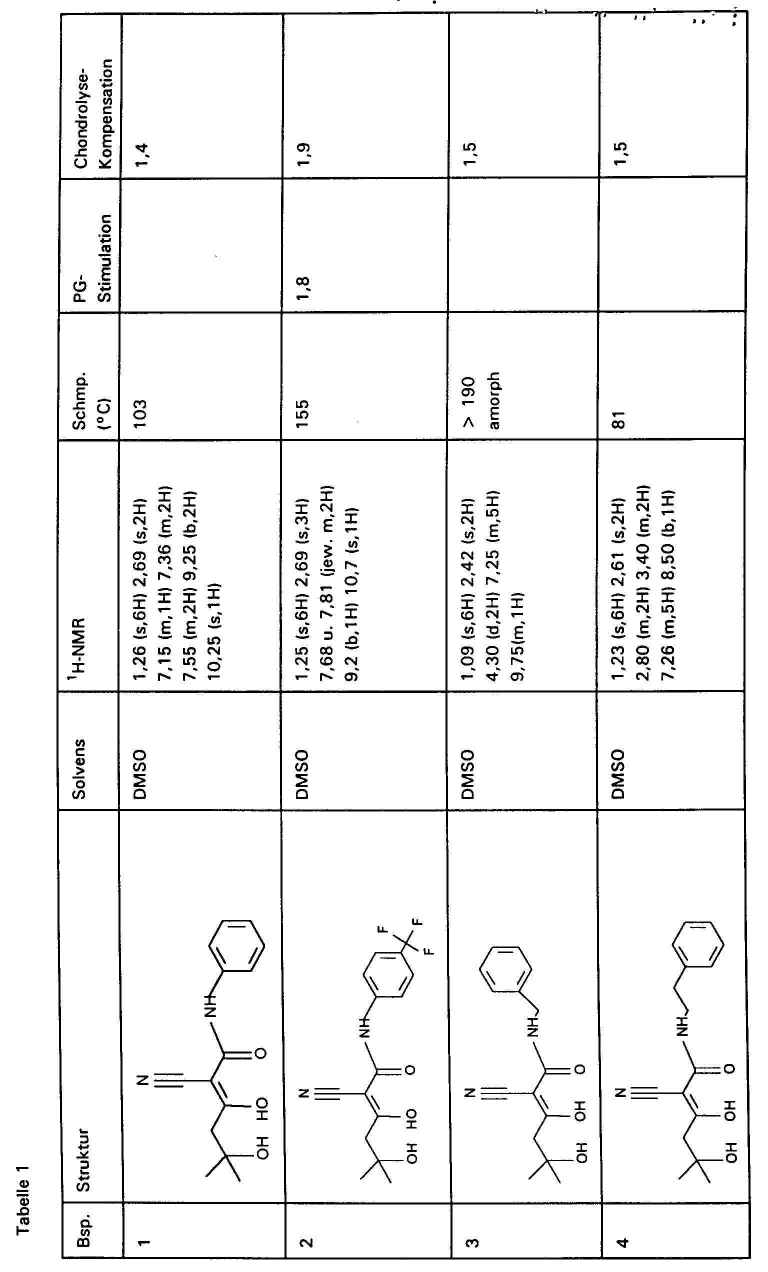

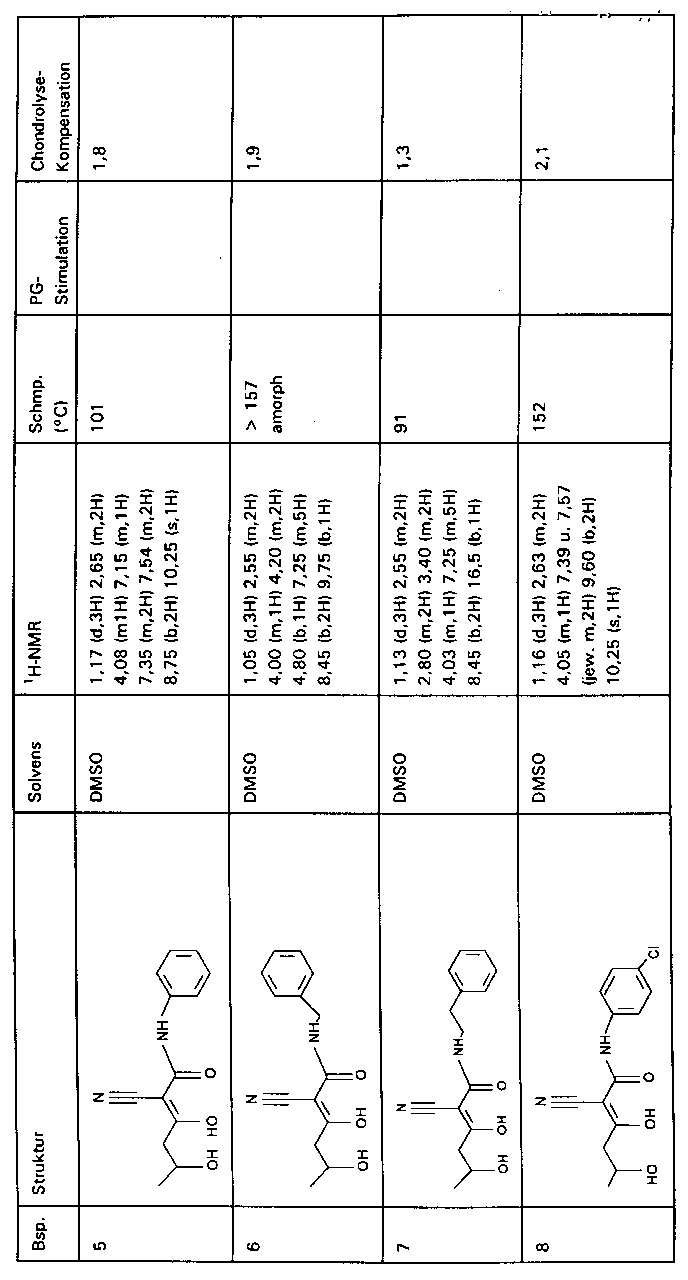

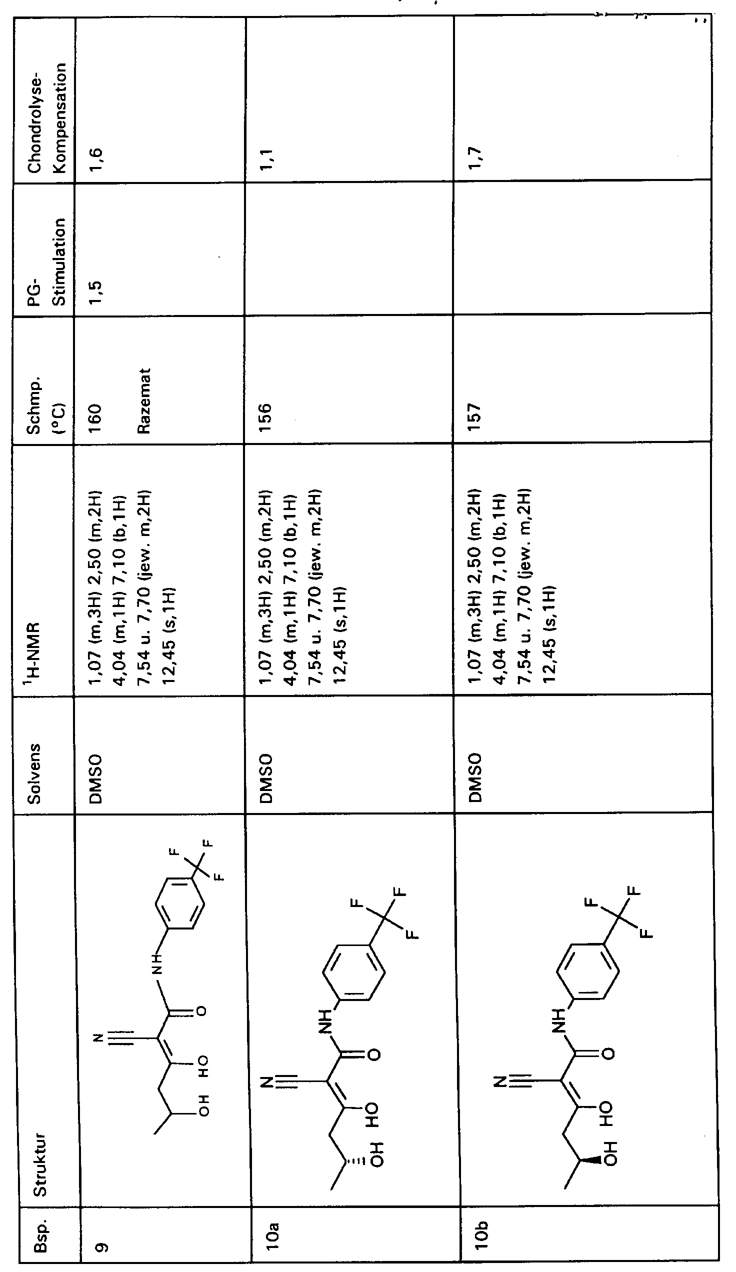

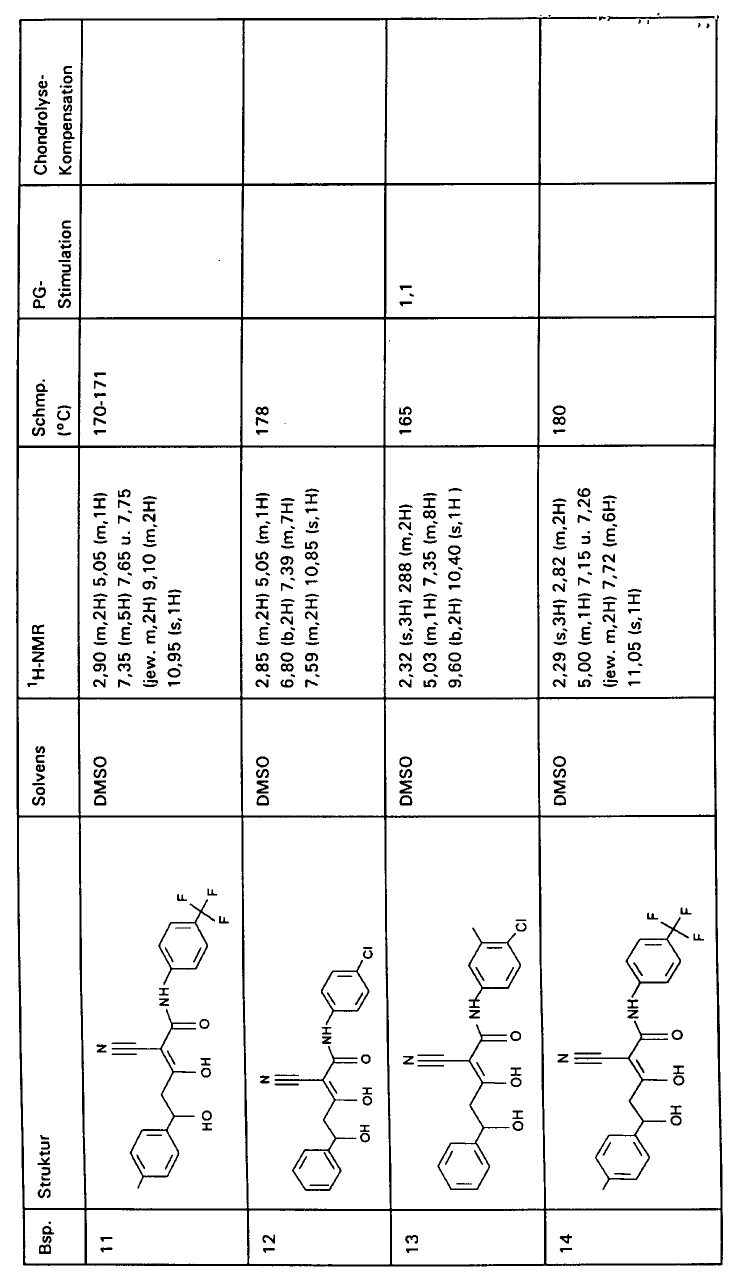

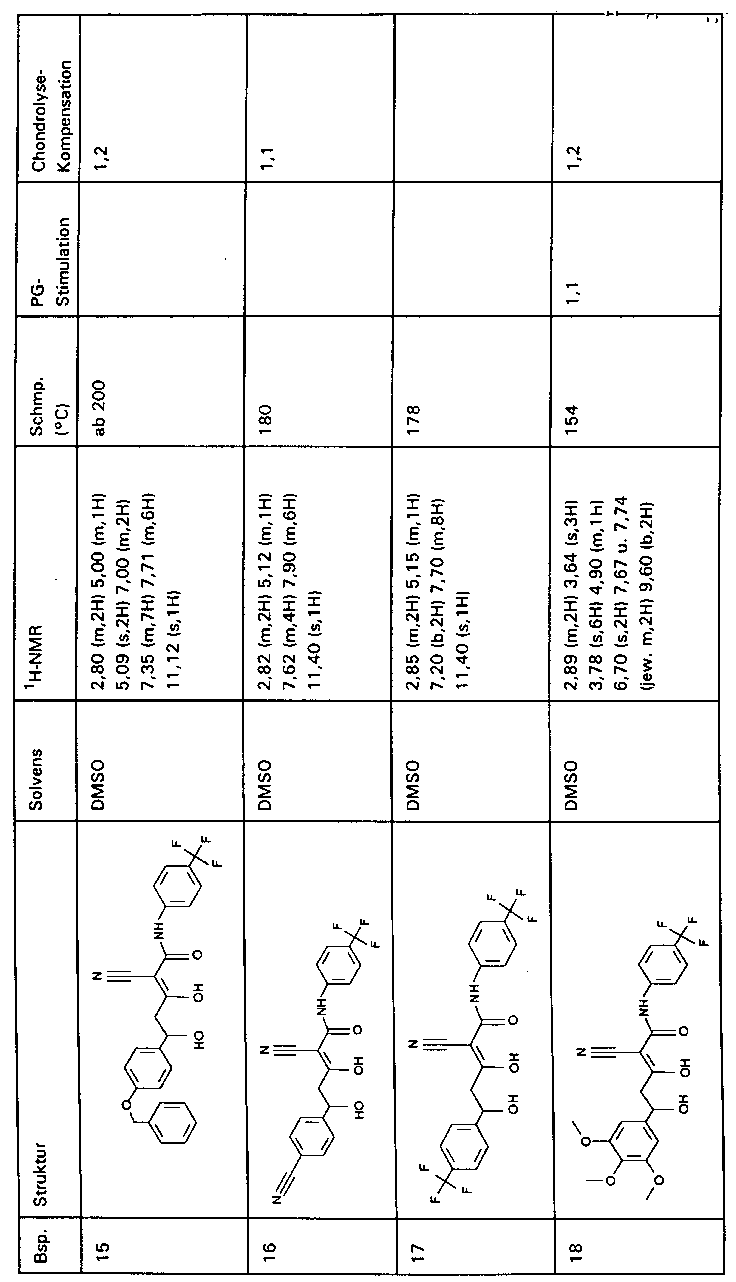

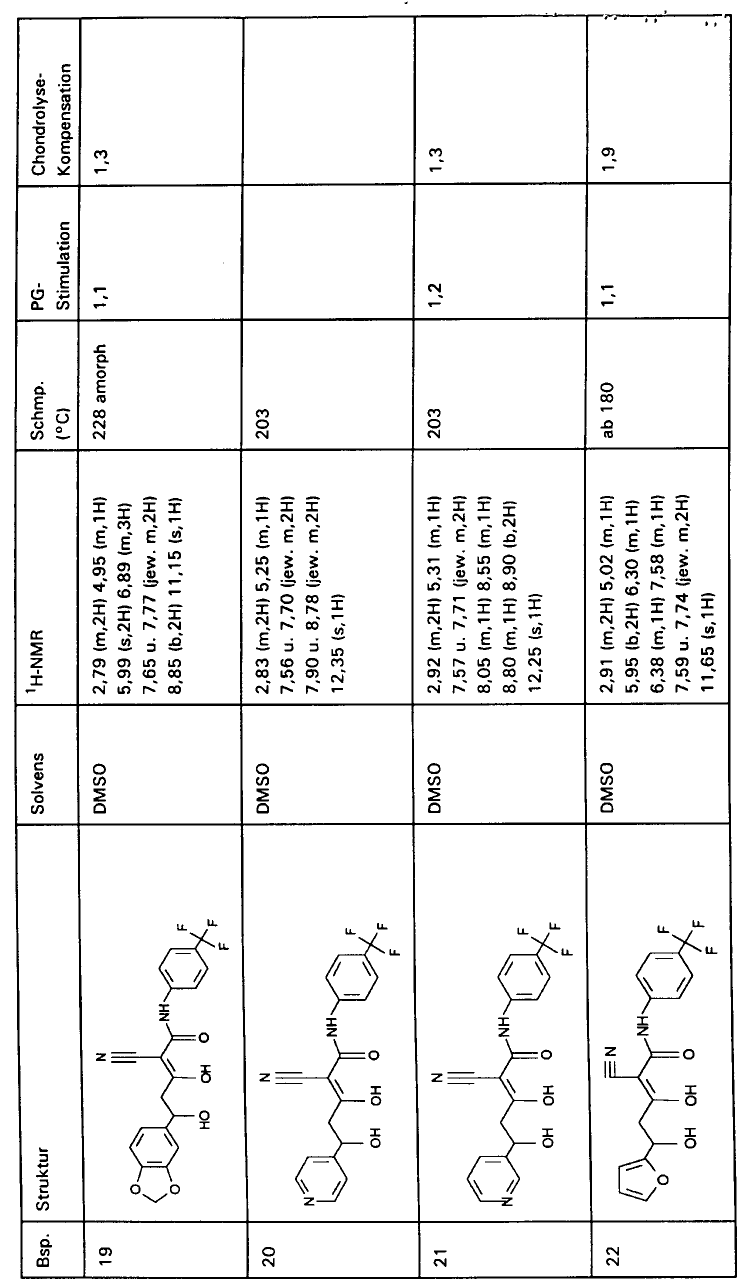

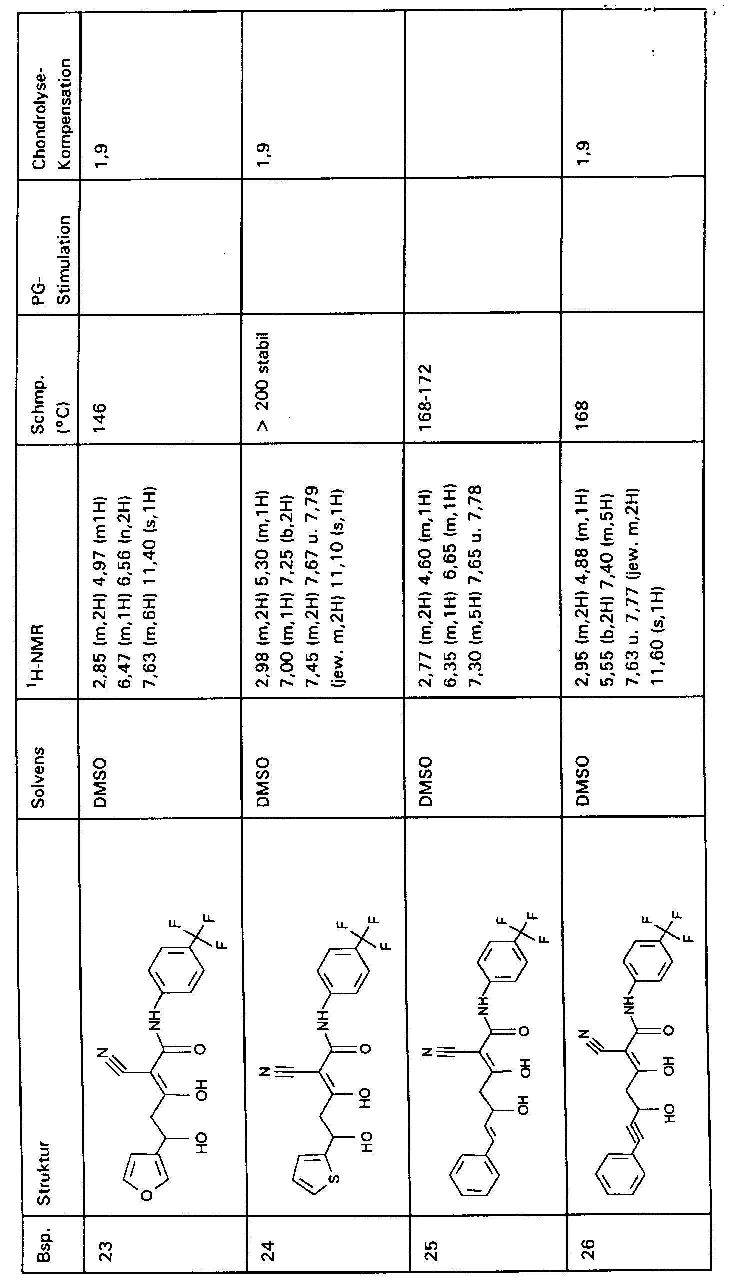

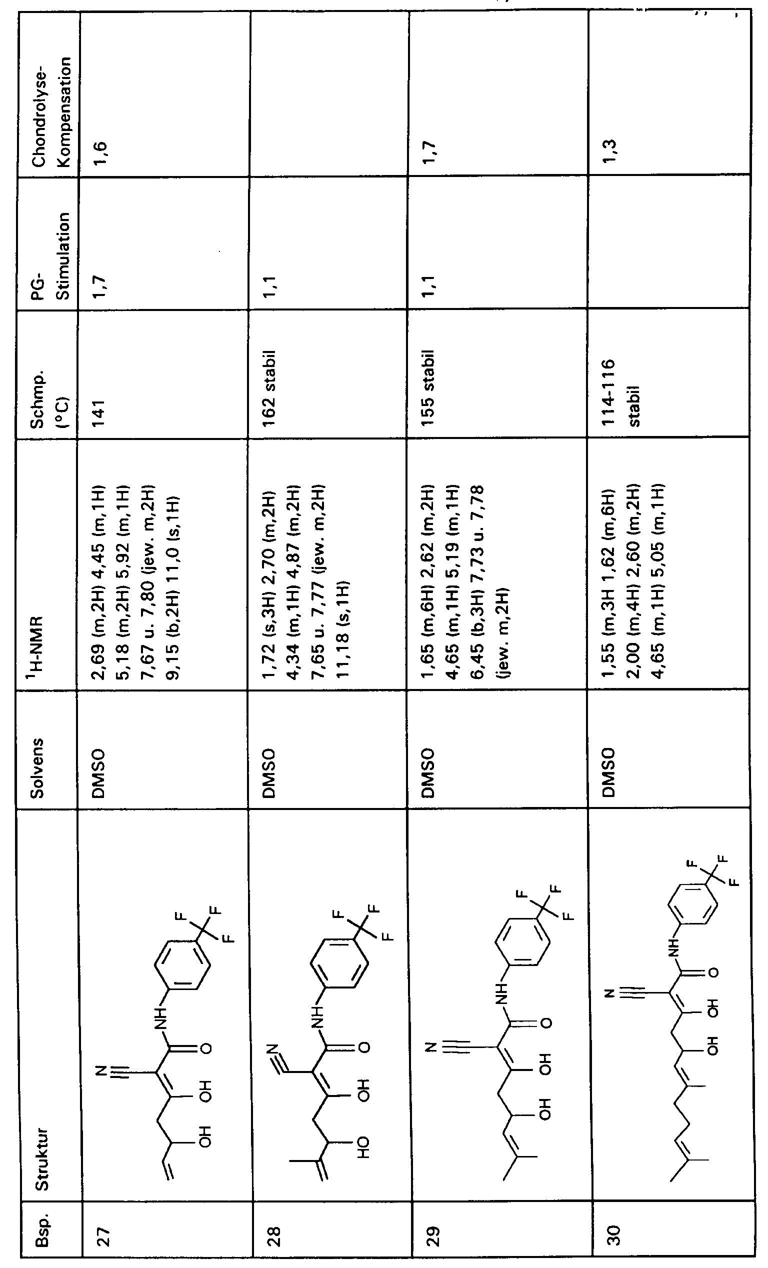

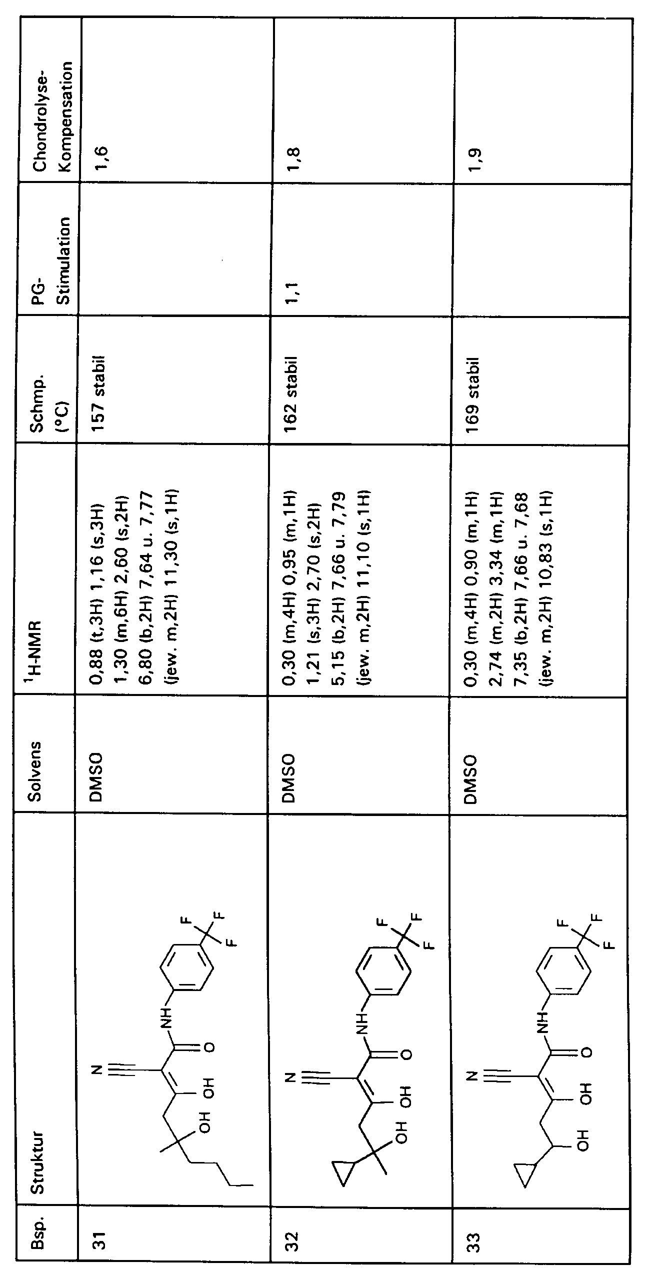

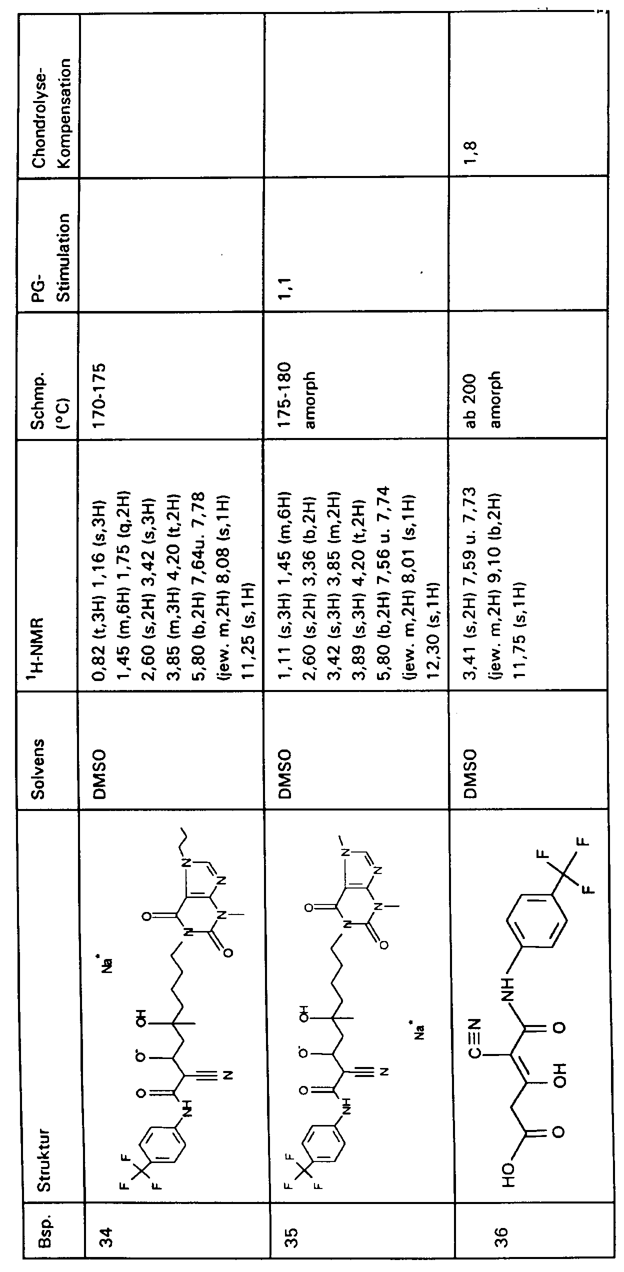

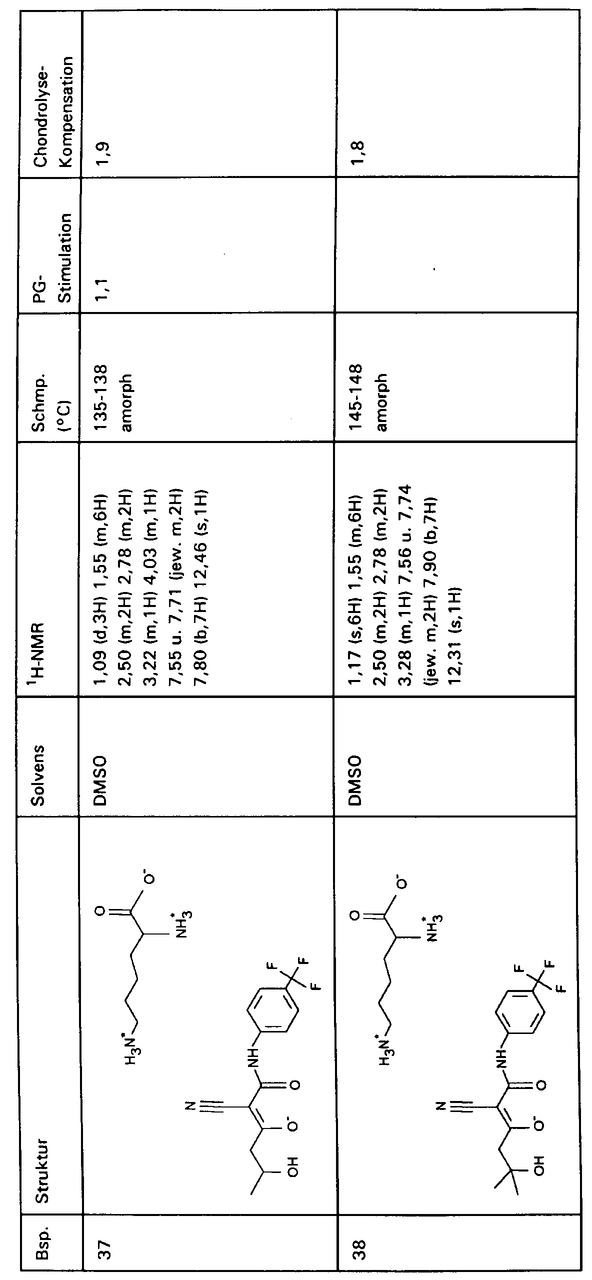

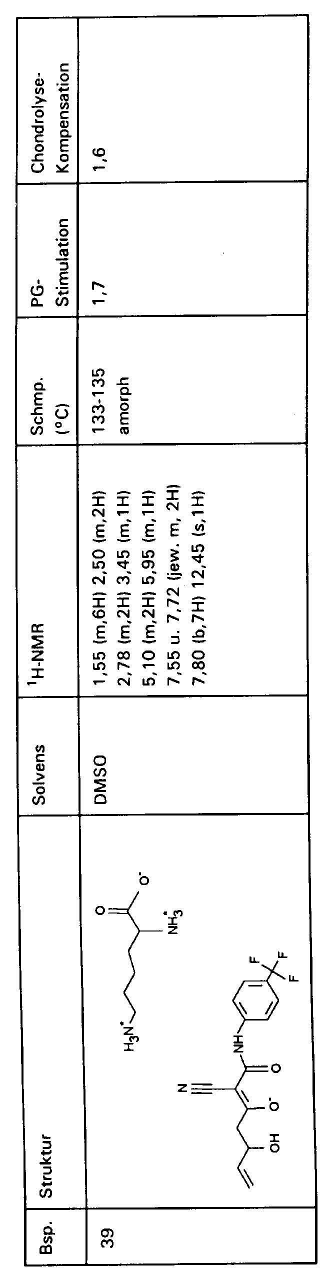

## Patentansprüche

1. Verbindung der Formel I, 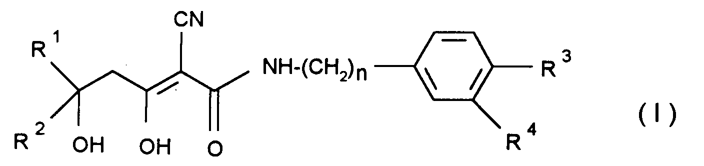 und/oder eine gegebenenfalls stereoisomere Form der Verbindung der Formel I und/oder ein physiologisch verträgliches Salz der Verbindung der Formel I, wobei
R¹ für
a) Wasserstoffatom oder
b) (C₁-C₄)-Alkyl steht,
R² für
a) (C₁-C₁₂)-Alkyl,
b) (C₂-C₁₂)-Alkenyl,
c) (C₂-C₁₂)-Alkinyl,
d) (C₃-C₇)-Cycloalkyl,
e) ein 4- bis 7-gliedriger heterozyklischer Rest mit 1 oder 2 Heteroatomen aus der Gruppe Sauerstoff, Stickstoff und Schwefel
f) Phenyl,
g) Phenyl, ein- bis dreifach substituiert durch
1) (C₁-C₄)-Alkyl,
2) Halogen,
3) -CN,
4) -CF₃,
5) -O-(C₁-C₄)-Alkyl,
6) -O-(C₁-C₄)-Alkyl substituiert durch Phenyl, oder
7) Methylendioxyl,
h) (C₁-C₆)-Alkyl, einmal substituiert durch
1) Phenyl oder
2) Rest der Formel II, 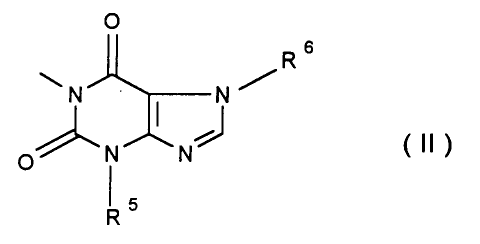 worin R⁵ und R⁶ unabhängig voneinander für
1) Wasserstoffatom oder
2) (C₁-C₄)-Alkyl stehen,
i) (C₂-C₆)-Alkenyl, einmal substituiert durch
1) Phenyl oder
2) Rest der Formel II, oder
k) (C₂-C₆)-Alkinyl, einmal substituiert durch
1) Phenyl oder
2) Rest der Formel II, steht, oder
l) R¹ und R² zusammen = O bedeuten,
R³ für
a) -CF₃,
b) -O-CF₃,
c) -S-CF₃,
d) -OH,
e) -NO₂,
f) Halogen,
g) Benzyl,
h) Phenyl,
i) -O-Phenyl,
j) Wasserstoffatom,
k) -CN,
l) -O-Phenyl, ein oder mehrfach substituiert durch
1) (C₁-C₄)-Alkyl,
2) Halogen,
3) -O-CF₃ oder
4) -O-CH₃, steht,
R⁴ für
a) (C₁-C₄)-Alkyl,
b) Halogen oder
c) Wasserstoffatom steht, und
n für die ganze Zahl Null, 1, 2, 3 oder 4 steht.

2. Verbindung der Formel I gemäß Anspruch 1 und/oder ein physiologisch verträgliches Salz der Verbindung der Formel I und/oder eine gegebenenfalls stereoisomere Form der Verbindung der Formel I, wobei
R¹ für
a) Wasserstoffatom oder
b) Methyl, steht,
R² für
a) (C₁-C₄)-Alkyl,
b) (C₂-C₇)-Alkenyl,
c) Cyclopropyl,
d) Phenyl,
e) ein 5- bis 6-gliedriger heterozyklischer Rest aus der Gruppe
1) Pyridin,
2) Furan oder
3) Thiophen,
f) Phenyl, ein- bis dreifach substituiert durch
1) Methyl,
2) -CN,
3) -CF₃,
4) -O-Methyl,
5) -O-Methylphenyl oder
6) Methylendioxy,
g) (C₁-C₄)-Alkyl substituiert durch den Rest der Formel II, worin R⁵ und R⁶ unabhängig voneinander für Methyl oder Propyl stehen,
h) Vinyl, substituiert durch Phenyl, oder
i) Ethinyl, substituiert durch Phenyl, steht, oder
k) R¹ und R² zusammen = O bedeuten,
R³ für
a) -CF₃,
b) -Cl oder
c) Methyl steht,
R⁴ für
a) Wasserstoffatom oder
b) Methyl steht, und
n für die ganze Zahl Null, 1 oder 2 steht.

3. Verbindung der Formel I gemäß Anspruch 1 oder 2 und/oder ein physiologisch verträgliches Salz der Verbindung der Formel I und/oder eine gegebenenfalls stereoisomere Form der Verbindung der Formel I, wobei
R¹ für Wasserstoffatom oder Methyl steht,
R² für
a) Methyl,
b) Butyl,
c) Vinyl,
d) 1-Methyl-ethenyl,
e) 2-Methyl-prop-enyl,
f) 2,6-Dimethyl-hepta-1,5-dienyl,
g) Cyclopropyl,
h) Phenyl,
i) Phenyl, ein- oder mehrfach substituiert durch
1) Methylendioxyl,
2) 4-Methyl,
3) Benzyloxy,
4) 4-Trifluormethyl,
5) Cyano oder
6) 3,4,5-Trimethoxy,
k) Furanyl,
l) Pyridyl,
m) Thiophenyl oder
n) Ethinylphenyl steht, oder
o) R¹ und R² zusammen = O bedeuten,
R³ für
a) -CF₃,
b) -Cl oder
c) Wasserstoffatom steht,
R⁴ für Wasserstoffatom oder Methyl steht und
n für die ganze Zahl Null, 1 oder 2 steht.

4. Verfahren zur Herstellung der Verbindung der Formel I gemäß der Ansprüche 1 bis 3, dadurch gekennzeichnet, daß man
A) ein entsprechend substituiertes 5-Methyl-isoxazol-3-carbonsäureamid oder -anilid bei tiefer Temperatur, bevorzugt bei -80 bis -40°C, mit einem Überschuß (ca. 3 Äquivalente) an starken organischen oder bevorzugt metallorganischen Basen wie Butyllithium, tert.-Butyllithium oder Lithiumdiisopropylamid in wasserfreien organischen Lösungsmitteln, wie Diethylether, Tetrahydrofuran oder tert.-Butylmethylether behandelt, wobei neben der baseninduzierten Ringöffnung des Isoxazolringes eine Deprotonierung der Methylgruppe stattfindet und diese deprotonierte Zwischenstufe mit elektrophilen Reagenzien wie Aldehyden, Ketonen oder Kohlendioxid nach entsprechender Aufarbeitung durch Ansäuern und Extraktion zu einer Addition an die Carbonylgruppe im Sinne einer C-C Verknüpfung führt oder
B) eine Carbonsäure, die mit weiteren funktionellen - oder in Form geschützter Vorstufen vorliegenden - Gruppen substituiert sein kann, in ein Säurehalogenid, vorzugsweise ein Säurechlorid, überführt, und mit der deprotonierten Form eines geeignet substituierten Cyanessigsäureamides oder -anilides im Sinne einer Kondensation umsetzt.

5. Arzneimittel, enthaltend eine wirksame Menge mindestens einer Verbindung der Formel I gemäß Anspruch 1 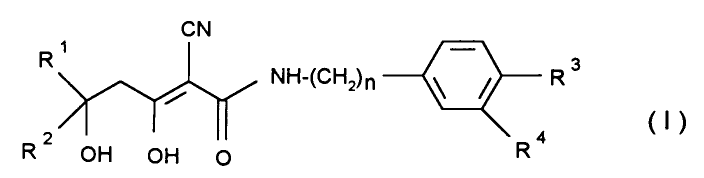 und/oder eines physiologisch verträglichen Salzes der Verbindung der Formel I und/oder eine gegebenenfalls stereoisomere Form der Verbindung der Formel I, wobei die Reste R¹, R², R³ und R⁴ wie in Anspruch 1 definiert sind, zusammen mit einem pharmazeutisch geeigneten und physiologisch verträglichen Trägerstoff, Zusatzstoff und/oder anderen Wirk- und Hilfsstoffen.

6. Verwendung von mindestens einer Verbindung der Formel I nach einem oder mehreren der Ansprüche 1 bis 3, zur Herstellung von Arzneimitteln zur Prophylaxe und Therapie von Erkrankungen oder Störungen, an deren Verlauf ein verstärkter Bindegewebs- und/oderKnorpel-Abbau beteiligt ist.

7. Verwendung gemäß Anspruch 6, dadurch gekennzeichnet, daß Erkrankungen des Bewegungsapparates wie entzündlich, immunologisch oder stoffwechselbedingte akute und chronische Arthritiden und Arthropathien, oder Myalgien, Störungen des Knochenstoffwechsels, sowie degenerative Gelenkserkrankungen wie Osteoarthrosen und Spondylosen, oder Knorpelschwund nach Gelenk-Trauma oder längerer Ruhigstellung behandelt werden.

8. Verwendung gemäß Anspruch 6, dadurch gekennzeichnet, daß Erkrankungen wie Kollagenosen und peridontalen Gewebsveränderungen behandelt werden.

9. Verfahren zur Herstellung eines Arzneimittels nach Anspruch 5, dadurch gekennzeichnet, daß man mindestens eine Verbindung der Formel I gemäß Anspruch 1 und/oder eine nach dem Verfahren gemäß Anspruch 4 erhaltene Verbindung mit physiologisch annehmbaren Hilfs- und Trägerstoffen und gegebenenfalls weiteren Zusatzstoffen und/oder anderen Wirkstoffen in eine geeignete Darreichungsform bringt.

## Claims

1. A compound of the formula I 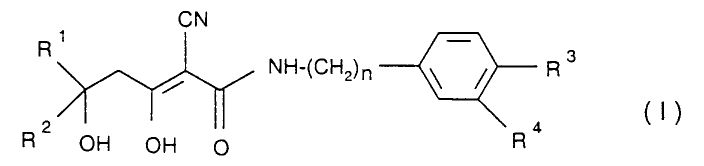 and/or an optionally stereoisomeric form of the compound of the formula I and/or a physiologically tolerable salt of the compound of the formula I, where
R¹ is
a) a hydrogen atom or
b) (C₁-C₄)-alkyl,
R² is
a) (C₁-C₁₂)-alkyl,
b) (C₂-C₁₂)-alkenyl,
c) (C₂-C₁₂)-alkynyl,
d) (C₃-C₇)-cycloalkyl,
e) a 4- to 7-membered heterocyclic radical having 1 or 2 heteroatoms from the group consisting of oxygen, nitrogen and sulfur
f) phenyl,
g) phenyl, mono- to trisubstituted by
1) (C₁-C₄)-alkyl,
2) halogen,
3) -CN,
4) -CF₃,
5) -O-(C₁-C₄)-alkyl,
6) -O-(C₁-C₄)-alkyl substituted by phenyl, or
7) methylenedioxyl,
h) (C₁-C₆)-alkyl, monosubstituted by
1) phenyl or
2) a radical of the formula II 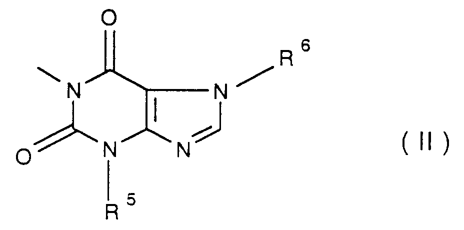
in which R⁵ and R⁶ independently of one another are
1) a hydrogen atom or
2) (C₁-C₄)-alkyl,
i) (C₂-C₆)-alkenyl, monosubstituted by
1) phenyl or
2) a radical of the formula II, or
k) (C₂-C₆)-alkynyl, monosubstituted by
1) phenyl or
2) a radical of the formula II, or
l) R¹ and R² together are = 0,
R³ is
a) -CF₃,
b) -O-CF₃,
c) -S-CF₃,
d) -OH,
e) -NO₂,
f) halogen,
g) benzyl,
h) phenyl,
i) -O-phenyl,
j) a hydrogen atom,
k) -CN,
l) -O-phenyl, mono- or polysubstituted by
1) (C₁-C₄)-alkyl,
2) halogen,
3) -O-CF₃ or
4) -O-CH₃,
R⁴ is
a) (C₁-C₄)-alkyl,
b) halogen, or
c) a hydrogen atom, and
n is the integer zero, 1, 2, 3 or 4.

2. A compound of the formula I as claimed in claim 1 and/or a physiologically tolerable salt of the compound of the formula I and/or an optionally stereoisomeric form of the compound of the formula I, where
R¹ is
a) a hydrogen atom or
b) methyl,
R² is
a) (C₁-C₄)-alkyl,
b) (C₂-C₇)-alkenyl,
c) cyclopropyl,
d) phenyl,
e) a 5- to 6-membered heterocyclic radical from the group consisting of
1) pyridine,
2) furan or
3) thiophene,
f) phenyl, mono- to trisubstituted by
1) methyl,
2) -CN,
3) -CF₃,
4) -O-methyl,
5) -O-methylphenyl or
6) methylenedioxyl,
g) (C₁-C₄)-alkyl substituted by the radical of the formula II, in which R⁵ and R⁶ independently of one another are methyl or propyl,
h) vinyl, substituted by phenyl, or
i) ethynyl, substituted by phenyl, or
k) R¹ and R² together are = 0,
R³ is
a) -CF₃,
b) -Cl or
c) methyl,
R⁴ is
a) a hydrogen atom or
b) methyl, and
n is the integer zero, 1 or 2.

3. A compound of the formula I as claimed in claim 1 or 2 and/or a physiologically tolerable salt of the compound of the formula I and/or an optionally stereoisomeric form of the compound of the formula I, where
R¹ is a hydrogen atom or methyl,
R² is
a) methyl
b) butyl,
c) vinyl,
d) 1-methylethenyl,
e) 2-methylpropenyl,
f) 2,6-dimethylhepta-1,5-dienyl,
g) cyclopropyl,
h) phenyl,
i) phenyl, mono- or polysubstituted by
1) methylenedioxyl,
2) 4-methyl,
3) benzyloxy,
4) 4-trifluoromethyl,
5) cyano or
6) 3,4,5-trimethoxy,
k) furanyl,
l) pyridyl,
m) thiophenyl or
n) ethynylphenyl, or
o) R¹ and R² together are= 0,
R³ is
a) -CF₃,
b) -Cl or
c) a hydrogen atom,
R⁴ is a hydrogen atom or methyl and
n is the integer zero, 1 or 2.

4. A process for the preparation of the compound of the formula I as claimed in claims 1 to 3, which comprises
A) treating an appropriately substituted 5-methylisoxazole-3-carboxamide or -anilide at low temperature, preferably at -80 to -40°C, with an excess (about 3 equivalents) of strong organic or preferably organometallic bases such as butyllithium, tert-butyllithium or lithium diisopropylamide in anhydrous organic solvents, such as diethyl ether, tetrahydrofuran or tert-butyl methyl ether, a deprotonation of the methyl group taking place in addition to the base-induced ring opening and this deprotonated intermediate leading with electrophilic reagents such as aldehydes, ketones or carbon dioxide, after appropriate working up by acidification and extraction, to an addition to the carbonyl group in the sense of a C-C linkage or
B) converting a carboxylic acid which can be substituted by further functional groups, or groups present in the form of protected precursors, into an acid halide, preferably an acid chloride, and reacting with the deprotonated form of a suitably substituted cyanoacetamide or -anilide in the sense of a condensation.

5. A pharmaceutical comprising an efficacious amount of at least one compound of the formula I as claimed in claim 1 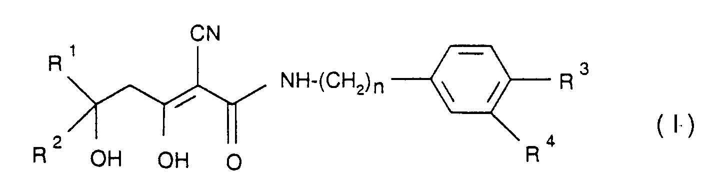 and/or a physiologically tolerable salt of the compound of the formula I and/or an optionally stereoisomeric form of the compound of the formula I, the radicals R¹, R², R³ and R⁴ being defined as in claim 1, together with a pharmaceutically suitable and physiologically tolerable excipient, additive and/or other active compounds and auxiliaries.

6. The use of at least one compound of the formula I as claimed in one or more of claims 1 to 3 for the production of pharmaceuticals for the prophylaxis and therapy of diseases or disorders whose course involves increased connective tissue and/or cartilage degradation.

7. The use as claimed in claim 6, wherein the diseases of the locomotory apparatus such as inflammatory, immunologically or metabolically related acute and chronic arthritides and arthropathies, or myalgias, disorders of the bone metabolism, as well as degenerative joint diseases such as osteoarthroses and spondyloses, or chondrolysis after joint trauma or relatively long immobilization, are treated.

8. The use as claimed in claim 6, wherein diseases such as collagenoses and peridontal tissue changes are treated.

9. A process for the production of a pharmaceutical as claimed in claim 5, which comprises bringing at least one compound of the formula I as claimed in claim 1 and/or a compound obtained by the process as claimed in claim 4 into a suitable administration form with physiologically acceptable auxiliaries and excipients and, if appropriate, further additives and/or other active compounds.

## Revendications

1. Composé de formule I 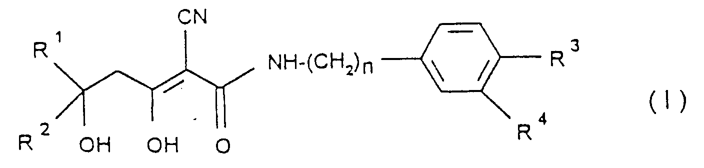 et/ou forme éventuellement stéréoisomère du composé de formule I et/ou sel physiologiquement acceptable du composé de formule I, dans laquelle
R¹ représente
a) un atome d'hydrogène ou
b) un groupe alkyle en C₁-C₄,
R² représente
a) un groupe alkyle en C₁-C₁₂,
b) un groupe alcényle en C₂-C₁₂,
c) un groupe alcynyle en C₂-C₁₂,
d) un groupe cycloalkyle en C₃-C₇,
e) un radical hétérocyclique à 4-7 chaînons, comportant 1 ou 2 hétéroatomes choisis parmi les atomes d'oxygène, d'azote et de soufre,
f) le groupe phényle,
g) un groupe phényle une à trois fois substitué par
1) un groupe alkyle en C₁-C₄,
2) un atome d'halogène,
3) -CN,
4) -CF₃,
5) un groupe -O-alkyle(C₁-C₄),
6) un groupe -O-alkyle(C₁-C₄) substitué par le groupe phényle, ou
7) le groupe méthylènedioxy,
h) un groupe alkyle en C₁-C₆ une fois substitué par
1) le groupe phényle ou
2) un radical de formule II, 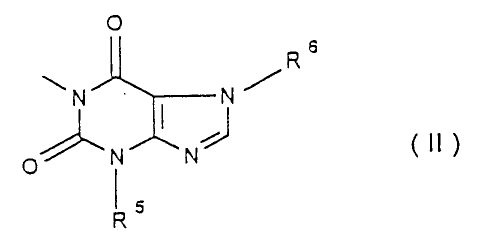
dans laquelle R⁵ et R⁶ représentent, indépendamment l'un de l'autre,
1) un atome d'hydrogène ou
2) un groupe alkyle en C₁-C₄,
i) un groupe alcényle en C₂-C₆ une fois substitué par
1) le groupe phényle ou
2) un radical de formule II, ou
k) un groupe alcynyle en C₂-C₆ une fois substitué par
1) le groupe phényle ou
2) un radical de formule II, ou
l) R¹ et R² représentent ensemble =O,
R³ représente
a) -CF₃,
b) -O-CF₃,
c) -S-CF₃,
d) -OH,
e) -NO₂,
f) un atome d'halogène,
g) le groupe benzyle,
h) le groupe phényle,
i) le groupe -O-phényle,
j) un atome d'hydrogène,
k) -CN,
l) un groupe -O-phényle une ou plusieurs fois substitué par
1) un groupe alkyle en C₁-C₄,
2) un atome d'halogène,
3) -O-CF₃ ou
4) -O-CH₃,
R⁴ représente
a) un groupe alkyle en C₁-C₄,
b) un atome d'halogène ou
c) un atome d'hydrogène, et
n représente le nombre entier zéro, 1, 2, 3 ou 4.

2. Composé de formule I selon la revendication 1 et/ou sel physiologiquement acceptable du composé de formule I et/ou forme éventuellement stéréoisomère du composé de formule I, dans laquelle
R¹ représente
a) un atome d'hydrogène ou
b) le groupe méthyle,
R² représente
a) un groupe alkyle en C₁-C₄,
b) un groupe alcényle en C₂-C₇,
c) le groupe cyclopropyle,
d) le groupe phényle,
e) un radical hétérocyclique à 5-6 chaînons, choisi dans l'ensemble constitué par
1) le cycle pyridine,
2) le cycle furanne et
3) le cycle thiophène,
f) un groupe phényle une à trois fois substitué par
1) le groupe méthyle,
2) -CN,
3) -CF₃,
4) le groupe -O-méthyle,
5) le groupe -O-méthylphényle ou
6) le groupe méthylènedioxy,
g) un groupe alkyle en C₁-C₄ substitué par le radical de formule II dans lequel R⁵ et R⁶ représentent, indépendamment l'un de l'autre, le groupe méthyle ou propyle,
h) un groupe vinyle substitué par le groupe phényle, ou
i) un groupe éthynyle substitué par le groupe phényle, ou
k) R¹ et R² représentent ensemble =O,
R³ représente
a) -CF₃,
b) -Cl ou
c) le groupe méthyle,
R⁴ représente
a) un atome d'hydrogène ou
b) le groupe méthyle, et
n représente le nombre entier zéro, 1 ou 2.

3. Composé de formule I selon la revendication 1 ou 2 et/ou sel physiologiquement acceptable du composé de formule I et/ou forme éventuellement stéréoisomère du composé de formule I, dans laquelle
R¹ représente un atome d'hydrogène ou le groupe méthyle,
R² représente
a) le groupe méthyle,
b) le groupe butyle,
c) le groupe vinyle,
d) le groupe 1-méthyl-éthényle,
e) le groupe 2-méthyl-propényle,
f) le groupe 2,6-diméthyl-hepta-1,5-diényle,
g) le groupe cyclopropyle,
h) le groupe phényle,
i) un groupe phényle une ou plusieurs fois substitué par
1) le groupe méthylènedioxy,
2) le groupe 4-méthyle,
3) le groupe benzyloxy,
4) le groupe 4-trifluorométhyle,
5) le groupe cyano ou
6) le groupe 3,4,5-triméthoxy,
k) le groupe furannyle,
l) le groupe pyridyle,
m) le groupe thiophényle ou
n) le groupe éthynylphényle, ou
o) R¹ et R² représentent ensemble =O,
R³ représente
a) -CF₃,
b) -Cl ou
c) un atome d'hydrogène,
R⁴ représente un atome d'hydrogène ou le groupe méthyle, et
n représente le nombre entier zéro, 1 ou 2.

4. Procédé pour la préparation du composé de formule I selon les revendications 1 à 3, caractérisé en ce que
A) on traite un 5-méthylisoxazole-3-carboxamide ou -anilide convenablement substitué, à basse température, de préférence à une température de -80 à -40°C, par un excès (environ 3 équivalents) de bases fortes organiques ou de préférence organométalliques, telles que le butyllithium, le tert-butyllithium ou le diisopropylamidure de lithium, dans des solvants organiques anhydres tels que l'oxyde d'éthyle, le tétrahydrofuranne ou l'éther tert-butylméthylique, une déprotonation du groupe méthyle ayant lieu en plus de l'ouverture de cycle induite par la base, et ce produit intermédiaire déprotoné conduit, après traitement final par acidification et extraction, à une addition avec des réactifs électrophiles tels que des aldéhydes, des cétones ou le dioxyde de carbone, sur le groupe carbonyle dans le sens d'une liaison C-C, ou
B) on convertit un acide carboxylique, qui peut être substitué par d'autres groupes fonctionnels - ou se trouvant sous forme de précurseurs protégés - en un halogénure d'acide, de préférence un chlorure d'acide, et on le fait réagir avec la forme déprotonée d'un cyanoacétamide ou -anilide convenablement substitué, dans le sens d'une condensation.

5. Médicament, contenant une quantité efficace d'au moins un composé de formule I selon la revendication 1, 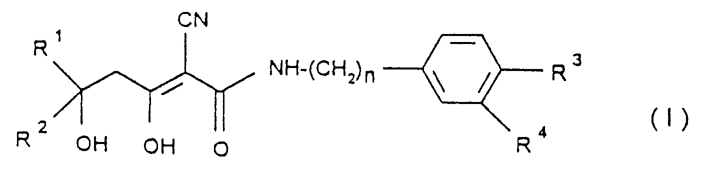 et/ou d'un sel physiologiquement acceptable du composé de formule I et/ou une forme éventuellement éventuellement stéréoisomère du composé de formule I, dans laquelle les radicaux R¹, R², R³ et R⁴ sont tels que définis dans la revendication 1, conjointement avec un véhicule, un additif, pharmaceutiquement appropriés et physiologiquement acceptables, et/ou d'autres adjuvants et substances actives.

6. Utilisation d'au moins un composé de formule I selon une ou plusieurs des revendications 1 à 3, pour la fabrication de médicaments destinés à la prophylaxie et au traitement de maladies ou de troubles à l'évolution desquels participe une dégradation accentuée du tissu conjonctif et/ou du cartilage.

7. Utilisation selon la revendication 6, caractérisée en ce que l'on traite des maladies de l'appareil locomoteur, telles que des arthrites et arthropathies aiguës et chroniques, d'origine inflammatoire, immunitaire ou métabolique, ou des myalgies, des troubles du métabolisme osseux, ainsi que des maladies articulaires dégénératives telles que les ostéo-arthroses et spondyloses, ou une atrophie du cartilage après un traumatisme articulaire ou une assez longue immobilisation.

8. Utilisation selon la revendication 6, caractérisée en ce que l'on traite des maladies telles que les collagénoses et les maladies tissulaires periodontiques.

9. Procédé pour la fabrication d'un médicament selon la revendication 5, caractérisé en ce que l'on met sous une forme d'administration appropriée au moins un composé de formule I selon la revendication 1 et/ou un composé obtenu conformément au procédé selon la revendication 4, avec des adjuvants et véhicules physiologiquement acceptables et éventuellement d'autres additifs et/ou d'autres substances actives.
